(19) Europäisches Patentamt
European Patent Office
Office européen des brevets

(11) Veröffentlichungsnummer: **0 559 238 B1**

(12) # EUROPÄISCHE PATENTSCHRIFT

(45) Veröffentlichungstag der Patentschrift: **03.05.95**

(51) Int. Cl.[6]: **C07C 255/23**

(21) Anmeldenummer: **93106367.1**

(22) Anmeldetag: **13.09.86**

(60) Veröffentlichungsnummer der früheren Anmeldung nach Art. 76 EPÜ: **0 217 206**

Die Akte enthält technische Angaben, die nach dem Eingang der Anmeldung eingereicht wurden und die nicht in dieser Patentschrift enthalten sind.

(54) **Cyancrotonsäureamidverbindung als Arzneimittel mit immunmodulierenden Eigenschaften.**

(30) Priorität: **27.09.85 DE 3534440**

(43) Veröffentlichungstag der Anmeldung: **08.09.93 Patentblatt 93/36**

(45) Bekanntmachung des Hinweises auf die Patenterteilung: **03.05.95 Patentblatt 95/18**

(84) Benannte Vertragsstaaten: **AT BE CH DE FR GB IT LI LU NL SE**

(56) Entgegenhaltungen:
**DE-A- 2 555 789**
**US-A- 4 061 767**

**ANN.REP.MED.CHEM. Bd. 18, 1983, Seiten 171 - 179 STRETCHER ET AL. 'Diseasemodifying anti-rheumatic drugs'**

**Int. J. Immunopharmac., vol. 7, no. 1, pp. 7-18, 1985 US-A-4284786**

(73) Patentinhaber: **HOECHST AKTIENGESELLSCHAFT**

**D-65926 Frankfurt (DE)**

(72) Erfinder: **Bartlett, Robert Ryder, Dr.**
**Schmittweg 23**
**W-6100 Darmstadt (DE)**
Erfinder: **Kämmerer, Friedrich-Johannes, Dr.**
**Am Gänsborn 3a**
**W-6203 Hochheim am Main (DE)**
Erfinder: **Schleyerbach, Rudolf, Dr.**
**Finkenweg 10**
**W-6238 Hofheim (Ts.) (DE)**

EP 0 559 238 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus der Europäischen Patentschrift 13 376 ist 5-Methylisoxazol-4-carbonsäure-(4-trifluormethyl)-anilid als antiphlogistisch bekannt. Dort sind ebenfalls Verfahren zur Herstellung dieser Verbindung beschrieben.

Es wurde nun gefunden, daß N-(4-Trifluormethylphenyl)-2-cyan-3-hydroxy-crotonsäureamid

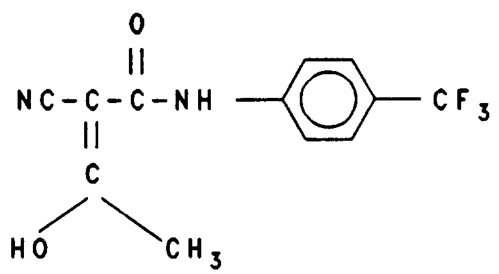

[Stecher und Carlson, Ann. Report Med. Chem. 18, 171-179 (1983)] solche immunmodulierenden Eigenschaften hat, daß sie sich als Arzneimittel gegen chronische Graft-versus-Host-Krankheiten (cGvH) sowie gegen Autoimmunerkrankungen, insbesondere systemischen Lupus erythematodes (SLE) eignet.

Gegenstand der Erfindung ist daher die Verwendung von N-(4-Trifluormethylphenyl]-2-cyan-3-hydroxycrotonsäureamid, wobei sie als solche oder in Form eines physiologisch verträglichen Salzes verwendet werden kann, zur Herstellung von Arzneimitteln gegen chronische Graft-versus-Host-Krankheiten sowie gegen Autoimmunerkrankungen, insbesondere systemischen Lupus erythematodes. Geeignete Salze sind z.B. Alkali-, Erdalkali- und Ammoniumsalze einschließlich solcher von physiologisch verträglichen organischen Ammoniumbasen.

A) Chronische Graft-vs-Host(cGvH)-Krankheiten

Bei Transplantationen kommt es häufiger zur Abstoßung des Transplantats. Die Transplantat-Wirt-Beziehung beschränkt sich jedoch nicht allein auf die Abstoßung durch den Wirtsorganismus; in bestimmten Fällen kann eine vom Transplantat ausgehende, gegen das Wirtsgewebe gerichtete Immunreaktion eintreten. Man unterscheidet zwischen einer akuten und einer chronischen Reaktion. Die akute Graft-vs-Host-Reaktion ist durch Milzvergrößerung, Leberschwellung, Hypertrophie der Lymphknoten, hämolytische Anämie, niedrigen Immunglobulin- und Komplementspiegel sowie verminderte Immunreaktivität gekennzeichnet. Die akut verlaufende Reaktion endet fast immer tödlich.

Daneben gibt es die chronische Form des Krankheitsverlaufs. Sie führt zu Lymphadenopathie, Immunkomplexglomerulonephritis und zur Bildung von vielen Antikörpern. Diese Verlaufsform ist milder als die akute Form und führt nicht innerhalb kurzer Zeit zum Tode. Symptome, die durch cGvH-Reaktion ausgelöst werden, ähneln sehr stark denen des systemischen Lupus erythematodes.

B) Systemischer Lupus erythematodes (SLE)

Der systemische Lupus erythematodes ist eine nicht-organspezifische Autoimmunkrankheit. Diese Erkrankung befällt zahlreiche Organe und verläuft chronisch mit akuten Schüben. Äußere Erscheinungsbilder des SLE sind Hautveränderungen des Gesichts. Meist sind weitere Hautareale sowie die Schleimhaut befallen. Auch Nephritiden, Endokarditiden, hämolytische Anämien, Leukopenien und Beteiligung des Zentralnervensystems werden beobachtet.

Zahlreiche immunologische Phänomene wurden bei SLE beobachtet. Antikörper gegen bestimmt körpereigene Antigene werden gebildet. Diese Antikörper, die sich bei SLE-Patienten nachweisen lassen, sind z.B. gegen die Basalmembran der Haut, gegen Lymphozyten, Erythrozyten und nukleare Antigene gerichtet.

In erster Linie bilden die gegen Doppelstrang-DNS (ds-DNS) gerichteten Antikörper mit diesen Komplexe, die sich zusammen mit Komplement auf kleinen Blutgefäßen ablagern und häufig zu Vasculitis führen. Diese Ablagerungen sind besonders gefährlich, wenn sie in den renalen Glomeruli vorkommen, da sie zu Glomerulonephritis und Nierenversagen führen. Die Häufigkeit der klinisch erfaßbaren Nierenbeteiligung wird in der Literatur mit 50 bis 80 % angegeben.

Für das Überleben der Kranken mit systemischen Lupus erythematodes sind Glukokortikoide und andere immunsuppressive Medikamente, z.B. Cyclophosphamid (CPA) von entscheidender Bedeutung. Ein spezifisches Mittel zur Heilung gibt es bisher nicht. Die Therapie zielte bisher darauf ab, eine akute Exazerbation zu verhindern oder zu überwinden und Rückfälle zu vermeiden. Zu diesem Zweck hat man die Patienten mit Glukokortikoiden und anderen Immunsuppressiva behandelt, die jedoch selbst gefährliche Nebenwirkungen haben.

Zur Erforschung des SLE gibt es verschiedene Tiermodelle. Bei einzelnen Mäusestämmen tritt ein spontaner SLE auf, wie bei Neuseeland-Mäusen oder bei MRL/1-Mäusen, das sind ursprünglich aus den Jackson Laboratories, Maine, USA, stammende und in eigener Tierhaltung unter spezifisch pathogenfreien (SPF-)Verhältnissen weiter gezüchtete Tiere. Es ist aber auch möglich, durch experimentellen Eingriff an nicht-autoimmunen Mäusen eine SLE-ähnliche Krankheit auszulösen.

Im folgenden beziehen sich die Mengenangaben mg/kg auf kg Körpergewicht; CMC bedeutet das Natriumsalz der Carboxymethylcellulose und o.P. "ohne Prüfsubstanz". In der Figur 3 ist N-(4-Trifluormethylphenyl)-2-cyan-3-hydroxycrotonsäureamid als Anilid 2 bezeichnet. Die Wirksubstanzen wurden oral im Gemisch mit CMC verabreicht. "N.K." bedeutet Negativ-Kontrolle.

C) Pharmakologische Prüfungen und Ergebnisse

1. Die chronische Graft-vs-Host-Krankheit

Dieses Modell wurde von GLEICHMANN et al. in verschiedenen Veröffentlichungen beschrieben. Der SLE wird dadurch induziert, daß eine GvH-Reaktion durch eine abnormale T-B-Zellkooperation ausgelöst wird [GLEICHMANN et al. Euro. J. Immunol. 12; 152-159 (1982)]. Die cGvH-Krankheit wurde durch zwei Injektionen von Milz- und Thymuszellen ausgelöst. Die Mäuse, gemäß GLEICHMANN et al. (DBA/2 x C57B1/6)F1-Generation, erhielten jeweils 70 x $10^6$ DBA/2-Zellen am 1. Tag und 8. Tag, die in 0,2 ml Kulturmedium intravenös injiziert wurden. Die Behandlung der Tiere erfolgte erstmals am 17. Tag nach der ersten Injektion der Donorzellen. Es wurde der Versuch 1.1 durchgeführt, wobei in diesem Experiment nur weibliche Tiere als Spender und als Empfänger benutzt wurden. Jedem Tier wurde 1 ml oral verabreicht, der jeweils die in dem Versuch 1.1 angegebene Menge Wirkstoff und daneben CMC in einer Konzentration von 100 mg/l enthielt.

Versuche

1.1) Es wurden vom 17. Tag an einmal täglich verabreicht CMC, o.P.,
1 mg/kg Indomethacin bzw.
2 mg/kg Prednisolon bzw.
20 mg/kg N-(4-Trifluormethylphenyl)-2-cyan-3-hydroxycrotonsäureamid
30 mg/kg N-(4-Trifluormethylphenyl)-2-cyan-3-hydroxycrotonsäureamid.
In jeder Gruppe waren 10-11 Tiere.
a) Proteinurie
Währen der Dauer des Versuchs (9-10 Wochen) wurde wöchentlich die Menge an Protein im Urin der Tiere festgestellt (Albu-sticks-Reagenzstäbchen, Ames Division, Miles Laboratories, Elkhard). Die Ergebnisse dieser Untersuchungen sind in der Figur 3 dargestellt.
b) Hemmungen des Graft-vs-Host-Index
Im Verlaufe einer cGvH-Krankheit wird die Milz infolge der Immunabwehr wesentlich vergrößert. Wenn man das Gewicht der Milz in Relation zum Körpergewicht des erkrankten Tieres setzt und dieses Verhältnis mit der entsprechenden Relation beim gesunden Tier vergleicht, erhält man den Graft-vs-Host-Index:

$$\text{GvH-Index} = \frac{\text{Milzgewicht X / Körpergewicht X}}{\text{Milzgewicht g / Körpergewicht g}}$$

wobei X = untersuchte (kranke) Tiere und g = gesunde Tiere.

Der GvH-Index ermöglicht die Feststellung der Krankheitsstärke: je größer der Index, desto stärker die Krankheit. Die Ergebnisse sind in Tabelle 1 wiedergegeben.

Tabelle 1

| Versuch | Substanz | GvH-Index | % Änderung |
|---|---|---|---|
| 1.1 | CMC, o.P. | 2,99 | 0 |
| 1.1 | 1 mg/kg Indomethacin je Tag | 3,44 | 115 |
| 1.1 | 2 mg/kg Prednisolon je Tag | 1,27 | - 58 |
| 1.1 | 20 mg/kg N-(4-Trifluormethylphenyl-2-cyan-3-hydroxycrotonsäureamid | 1,99 | - 33 |
| 1.1 | 30 mg/kg N-(4-Trifluormethylphenyl-2-cyan-3-hydroxycrotonsäureamid | 1,27 | - 56 |

Die Ergebnisse der vorstehenden Untersuchungen zeigen, daß N-(4-Trifluormethylphenyl)-2-cyan-3-hydroxycrotonsäureamid die Entstehung von cGvH- und SLE-Krankheiten in Mäusen hemmen.

Durch diese Substanz wird

1) die Entstehung einer Glomerulonephritis bei beiden Krankheiten verhindert; dies konnte durch die verminderte Proteinausscheidung im Urin und durch histologische Untersuchungen an Nieren gezeigt werden;

2) der Anti-ds-DNS-Antikörpertiter herabgesetzt;

3) der GvH-Index vermindert;

4) die verminderte T-Lymphozyten-Proliferation dosisabhängig verbessert.

N-(4-Trifluormethylphenyl)-2-cyan-3-hydroxycrotonsäureamid zeigt Vorteile gegenüber Immunsuppressiva wie Cyclophosphamid oder Glukokortikoiden, da sie keine allgemeine Suppression des Immunsystems verursacht und sogar die Wiederherstellung der verminderten T-Zellenfunktion ermöglicht. Um so überraschender ist die Tatsache, daß sie gegen die chronische GvH-Krankheiten und die SLE gut wirksam ist.

N-(4-Trifluormethylphenyl)-2-cyan-3-hydroxycrotonsäureamid kann entweder allein, gegebenenfalls in Form von Mikrokapseln, oder vermischt mit üblichen physiologisch verträglichen Trägerstoffen, Verdünnungsmitteln und/oder Konstituentien verabreicht werden. Die Mittel könne oral, rektal, intravenös oder parenteral verabreicht werden, wobei die orale oder rektale Anwendung bevorzugt ist. Geeignete feste oder flüssige galenische Zubereitungsformen sind beispielsweise Granulate, Pulver, Tabletten, Dragees, Kapseln, Zäpfchen, Sirupe, Emulsionen, Suspensionen, Aerosole, Tropfen oder injizierbare Lösungen in Ampullenform, wobei auch die Trockenampulle als eine spezielle Zubereitungsform eingeschlossen ist, sowie Präparate mit protrahierter Wirkstoff-Freigabe, bei deren Herstellung gewöhnlich Hilfsmittel, wie Trägerstoffe, Spreng-, Binde-, Überzugs-, Quellungs-, Gleit- oder Schmiermittel, Geschmacksstoffe, Süßungsmittel, Puffersubstanzen, Antioxidantien und/oder Lösungsvermittler, Verwendung finden. Häufig verwendete Hilfsstoffe sind z.B. Magnesium- oder Calciumcarbonat, Calciumphosphate, Titandioxid, Mannit, Laktose und andere Zucker, Talkum, Milcheiweiß, Gelatine, Stärke, Vitamine, Cellulose und ihre Derivate, tierische und pflanzliche Öle, Polyäthylenglykole und physiologisch unbedenkliche Lösungsmittel, wie steriles Wasser, Alkohle, Glycerin und andere mehrwertige Alkohole.

Vorzugsweise werden die pharmazeutischen Präparate in Dosierungseinheiten hergestellt und verabreicht, wobei jede Einheit als aktiven Bestandteil eine bestimmte Dosis von N-(4-Trifluormethylphenyl)2-cyan-3-hydroxycrotonsäureamid enthält. Bei festen Dosierungseinheiten, wie Tabletten, Kapseln und Suppositorien, kann diese Dosis von 10 bis 200 mg, bevorzugt jedoch 50 bis 100 mg, bei Injektionslösungen in Ampullenform (intravenös), insbesondere auf Basis von N-(4-Trifluormethylphenyl)2-cyan-3-hydroxycrotonsäureamid bwz. eines Salzes davon, 1 bis 30 mg, vorzugsweise 5 bis 10 mg und bei rektaler Verabreichung 50 bis 300 mg, vorzugsweise 100 bis 200 mg betragen.

Für die Behandlung eines erwachsenen Patienten sind am Menschen Tagesdosen von 50 bis 200 mg Wirkstoff bei oraler Verabreichung, von 10 bis 30 mg bei intravenöser Applikation und von 100 bis 300 mg bei rektaler Applikation indiziert. Unter Umständen können jedoch auch höhere oder niedrigere Tagesdosen empfehlenswert sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Eine weitere Anwendung der Verbindungen besteht in der Kombination mit anderen geeigneten Wirkstoffen, beispielsweise Antiuricopathika, Thrombocytenaggregationshemmern, Analgetika und steroidalen oder nichtsteroidalen Antiphlogistika.

**Patentansprüche**

1. Verbindung der Formel

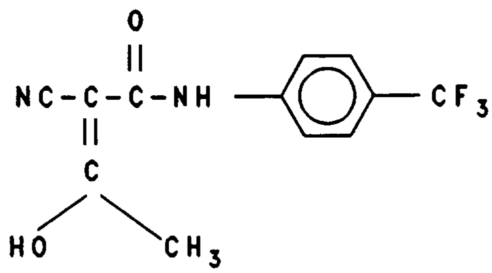

wobei die Verbindung als solche oder in Form eines physiologisch vertäglichen Salzes vorliegt.

2. Arzneimittel, enthaltend eine wirksame Menge der Verbindung gemäß Anspruch 1, wobei die Verbindung als solche oder in der Form eines Salzes vorliegt.

**Claims**

1. A compound of the formula

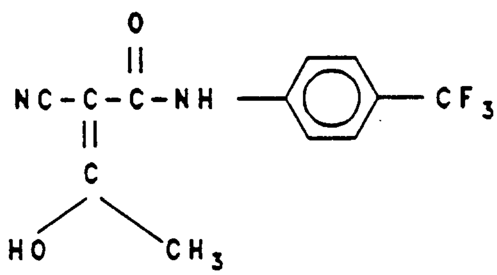

the compound being present as such or in the form of a physiologically tolerated salt.

2. A medicament containing an effective amount of the compound, as claimed in claim 1 the compound being present as such or in the form of a salt.

**Revendications**

1. Composé de formule

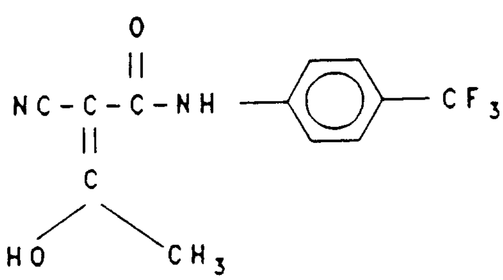

le composé se trouvant tel quel ou sous forme d'un sel physiologiquement acceptable.

2. Médicament contenant une quantité efficace du composé selon la revendication 1, le composé se trouvant tel quel ou sous la forme d'un sel.

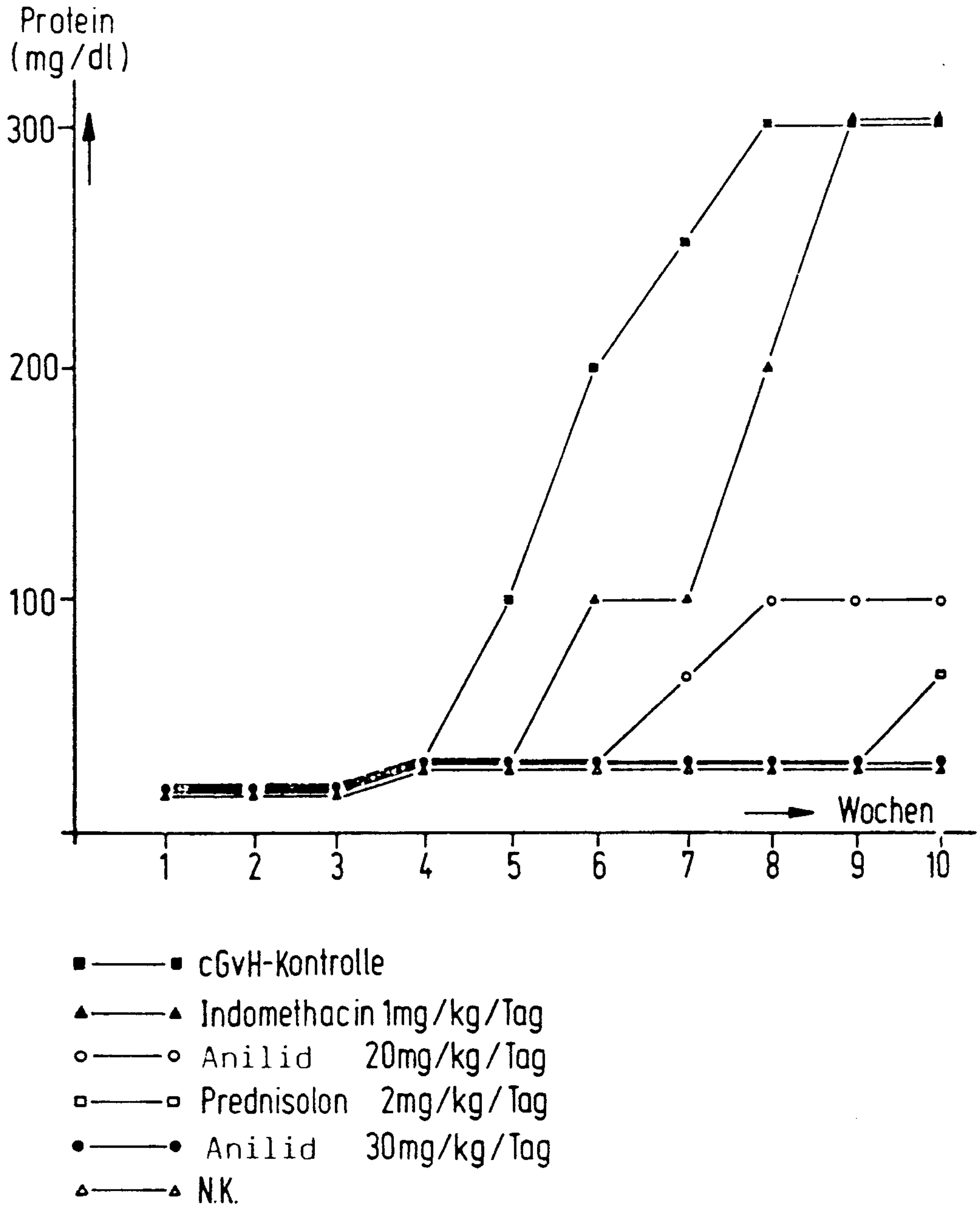
Protein
(mg/dl)
300
200
100
Wochen
1
2
3
4
5
6
7
8
9
10
cGvH-Kontrolle
Indomethacin 1mg/kg/Tag
Anilid 20mg/kg/Tag
Prednisolon 2mg/kg/Tag
Anilid 30mg/kg/Tag
N.K.